# EUROPÄISCHE PATENTSCHRIFT

(11) **EP 0 539 923 B1**
(45) Veröffentlichungstag und Bekanntmachung des Hinweises auf die Patenterteilung: **17.01.1996**
(21) Anmeldenummer: 92118352.1
(22) Anmeldetag: 27.10.1992
(51) Int. Cl.: G01N 33/535, C12Q 1/28

(54) **Lagerfähige Peroxidase-Konjugat-Lösungen**
Storage stable peroxidase conjugate solutions
Composé liquide conjugué de peroxidase stable au stockage

(30) Priorität: 28.10.1991 DE 4135542
(43) Veröffentlichungstag der Anmeldung: 05.05.1993
(73) Patentinhaber: BOEHRINGER MANNHEIM GMBH, D-68298 Mannheim (DE)
(72) Erfinder: Franken, Norbert, Dr., W-8130 Starnberg (DE); Hoyle, Nicholas, Dr., W-8132 Tutzing (DE); Pappert, Gunter, Dr., W-8132 Tutzing (DE)
(74) Vertreter: Huber, Bernhard, Dipl.-Chem.

(56) Entgegenhaltungen:
- EP-A- 0 080 304
- EP-A- 0 361 088
- EP-A- 0 426 552
- EP-A- 0 456 309
- EP-A- 0 487 301

## Beschreibung

In der DD 237327 werden zur Stabilisierung von POD-Konjugaten Proteine, heterocyclische Azine und Polyalkohole in einem Medium mit einem pH-Wert zwischen 6,0 und 7,5 beschrieben. In der EP-A 0 303 062 werden zur Stabilisierung von POD-Konjugaten hydrolisiertes Hühneralbumin und/oder Kälberserum, Polyvinylpyrrolidon und/oder Calciumpropionat, ANS (Anilinonaphthylsulfonsäure) in Trispuffer pH 7,2 beschrieben; die EP-A 0 197 447 beschreibt Aminopyrin, beispielsweise in Phosphat-, Citrat-, Boratpuffer; aus der DE-A 31 00 076 ist ein ANS und Serumprotein enthaltendes Medium bekannt; die EP-A 0 070 992 beschreibt ein 4-Aminoantipyrin, ANS und Serumprotein enthaltendes Medium; die EP-A-O 426552 Beschreibt die Verwendung von Harnsäure als stabilisierendes Agens, die US-A 4,169,012 beschreibt die Verwendung polyvalenter Ionen der Gruppen 2 und 3 des periodischen Systems. Mit diesen Zusätzen können POD-Konjugate in Lösung jedoch nur begrenzte Zeit stabilisiert werden. Bereits nach wenigen Tagen wird eine deutliche Abnahme der POD-Aktivität bzw. der Stabilität im immunologischen Test beobachtet.

Aufgabe der Erfindung ist die Bereitstellung einer bei Raumtemperatur über längere Zeit lager fähigen POD-Konjugat-Lösung mit verbesserter Stabilität, die auf einfache und reproduzierbare Weise herstellbar ist.

Gelöst wird diese Aufgabe durch eine lagerfähige POD-Konjugatlösung, gekennzeichnet durch einen pH-Wert von 5,0 bis 6,5, eingestellt durch eine organische Puffersubstanz einer Konzentration von 40 bis 100 mmol/l und 0,05 bis 1,0 mol/l Magnesiumsaspartat.

Als POD-Konjugat werden vorzugsweise die in für immunologische Bestimmungsverfahren eingesetzten Konjugate aus POD als Marker und einer bindefähigen, insbesondere immunologisch bindefähigen Substanz verwendet. Als bindefähiger Konjugatpartner des POD kommen vor allem Antikörper, Fragmente derselben, Antigene, Haptene, Biotin und Streptavidin in Betracht.

Als organische Puffersubstanz werden erfindungsgemäß insbesondere Good-Puffer, Citrat-Puffer und/oder 2-(N-Morpholino)- ethansulfonsäure (MES) eingesetzt. Vorzugsweise wird MES verwendet.

Aufgrund der aufeinander abgestimmten pH-Werte, der Konzentration der Puffersubstanzen und der Gegenwart von Magnesiumaspartat werden mit den erfindungsgemäßen POD-Konjugatlösungen hervorragende Stabilitätsergebnisse erzielt.

Vorzugsweise enthalten die erfindungsgemäßen Lösungen zusätzlich Stabilisatoren, Konservierungsmittel und/oder inerte Proteine und zwar insbesondere 0 bis 0,4 g/l Stabilisatoren, 0 bis 1 g/l Konservierungsmittel und/oder 0 bis 20 g/l inertes Protein.

Inerte Proteine (Stützproteine) sind z.B. Albumine, Casein, Immunglobuline; erfindungsgemäß verwendete Stabilisatoren sind z.B. ANS, Phenol oder Dimethylaminoantipyrin; und erfindungsgemäß verwendete Konservierungsmittel sind z.B. Kathon, Oxipyrion, Methylisothiazolon.

Die erfindungsgemäßen Lösungen besitzen hervorragende Stabilitäten. Für verschiedene POD-Konjugate (z.B. MAK-POD, Hapten-POD) beträgt nacht 3-wöchiger Lagerung bei 35°C die Peroxidase-Aktivität nöch 80 bis 99 % des Ausgangswerts.

Die nachfolgenden Beispiele sollen nun die Erfindung näher erläutern, ohne sie darauf zu beschränken. Wenn nicht anders angegeben, beziehen sich die Prozentangaben und Teile auf das Gewicht, Temperaturangaben auf die Celsius-Skala.

### Beispiel 1

### Durchführung eines immunologischen Bestimmungsverfahrens

Der Test wird nach dem ELISA-Testprinzip als Ein-Schritt-Sandwich-Test mittels festphasengebundenem Streptavidin, einem biotinylierten, monoklonalen Antikörper (MAK-BI) und einem Peroxidase-markierten zweiten monoklonalen Antikörper (MAK-POD) durchgeführt.

In ein mit Streptavidin beschichtetes Polystyrolröhrchen (Herstellung nach EP-A 0 269 092) werden 100 µl Probe und 1000 µl Arbeitslösung zugegeben.

### Zusammensetzung der POD-Lagerlösung:

MAK-POD (POD-Aktivität: 20 U/ml)
40 mmol/l Puffer (4-Morpholinethansulfonsäure, Phosphatpuffer oder Citratpuffer) (pH-Wert entsprechend Tabelle 1),
0,1 mol/l Magnesiumaspartat
0,2 g/l 4-Dimethylaminoantipyrin
0,1 g/l Oxypyrion
10 g/l Rinderimmunglobulin

### Zusammensetzung der Arbeitslösung:

1,5 µg/ml MAK-Bi
1/100stel POD-Lagerlösung

Es wird 1 Stunde bei Raumtemperatur inkubiert, zweimal mit 0,9 % NaCl-Lösung gewaschen und 1000 µl Substratlösung zugegeben.
Substratlösung:
100 mmol/l Phosphat-Citrat-Puffer pH 5,0
1,47 mmol/l Natriumperborat
9,1 mmol/l 2,2'-Azino-di-[3-ethylbenzthiazolin-sulfonsäure(6)]diammoniumsalz (ABTS®)

Es wird 30 Minuten inkubiert und die gebildete Farbe bei 420 nm fotometrisch bestimmt.

### Beispiel 2

### Bestimmung einer Peroxidaseaktivität in MAK-POD-Konjugaten

2,9 ml Substratlösung und 0,1 ml verdünnte Probe (MAK-POD) werden in eine Küvette mit 1 cm Schichtdicke zusammengegeben und nach Mischung die Absorptionsänderung bei Raumtemperatur über 5 Minuten bei 405 nm verfolgt.

Es wird ein Mittelwert der Extinktionsänderung pro Minute ermittelt. Die POD-Aktivität wird über folgende Formel ermittelt:
Aktivität = 0,8152 x A/min (U/ml Probelösung)

### Beispiel 3

### Stabilität von Anti-TSH-Antikörper-POD-Konjugat in verschiedenen Pufferlösungen

Anti-TSH-Antikörper-POD-Konjugat wurde in POD-Lagerlösung (ohne Zusatz von MAK-BI) bei verschiedenen pH-Werten in verschiedenen Puffersubstanzen 3 Wochen bei 4°C bzw. 35°C gelagert.

Die Funktionsfähigkeit des Konjugats wurde nach dem in Beispiel 1 beschriebenen Verfahren in Gegenwart von Puffer und Magnesiumaspartat überprüft. Ebenso wurde die POD-Aktivität nach Beispiel 2 überprüft.

### Die Ergebnisse zeigt Tabelle 1:

**Tabelle 1**

| Pufferion, pH | Aktivität nach Lagerung von | | | |
|---|---|---|---|---|
| | 3 Wochen 4°C | | 3 Wochen 35°C | |
| | Immun- | POD- | Immun- | POD-Aktivität |
| Acetat, pH 6,0 | 100 % | 100 % | 87 % | 90 % |
| Phosphat, pH 6,0 | 100 % | 100 % | 78 % | 100 % |
| MES, pH 6,5 | 100 % | 100 % | 87 % | 100 % |
| MES, pH 6,0 | 100 % | 100 % | 87 % | 100 % |
| MES, pH 5,0 | 100 % | 100 % | 82 % | 100 % |
| MES, pH 4,0 | 100 % | 100 % | 43 % | n.b. |
| Phosphat, pH 8,0 | 100 % | 100 % | 62 % | 100 % |

Tabelle 1 zeigt, daß zwar die POD-Aktivität nach Lagerung noch gegeben ist bei 3-wöchiger Lagerung bei 35°C, jedoch nur dann geringe Änderungen in der Richtigkeit sich ergeben, wenn der pH-Wert des Puffers zwischen 5 und 6,5 liegt.

### Beispiel 4

### Einfluß von Magnesiumaspartat

Wie in Beispiel 3 beschrieben, wird Anti-TSH-Antikörper-POD-Konjugat (Aktivität 20 U/ml in 40 mmol/ml 4-MES-Puffer bei pH 6,0) in Gegenwart von unterschiedlichen Magnesiumaspartatkonzentrationen gelagert.

Die Stabilität der Lösungen wurde nach 3 Wochen Lagerung in einem Test nach Beispiel 1 überprüft. Die Ergebnisse zeigt Tabelle 2.

**Tabelle 2**

| | Aktivität nach Lagerung von | |
|---|---|---|
| Magnesiumaspartat Konzentration | 3 Wochen 4°C | 3 Wochen 35°C |
| 0,00 mol/l | 100 % | 64 % |
| 0,05 mol/l | 100 % | 84 % |
| 0,10 mol/l | 100 % | 77 % |
| 0,25 mol/l | 100 % | 81 % |

Tabelle 2 zeigt, daß Magnesiumaspartat einen positiven Einfluß auf die Funktionsfähigkeit des Anti-TSH-Antikörperkonjugats hat.

### Beispiel 5

Einfluß von Pufferlösungen mit einem pH-Wert zwischen 5,5 und 6,0 auf die Stabilität von verschiedenen POD-Konjugaten.

Die in der Tabelle 3 genannten POD-Hapten bzw. AntikörperKonjugate wurden mit einer durchschnittlichen enzymatischen Aktivität von 0,4 bis 20 U/ml in einer Lösung aus 40 mmol/l 4-MES (pH 5,0 bis 6,0) 0,1 g/l Oxypyrion und 10 g/l Rinderimmunglobulin bei 3 Wochen bei 4°C bzw. 35°C gelagert. Anschließend wird ein entsprechender Funktionstest für das Antigen analog zu Beispiel 1 durchgeführt. Die Ergebnisse zeigt Tabelle 3.

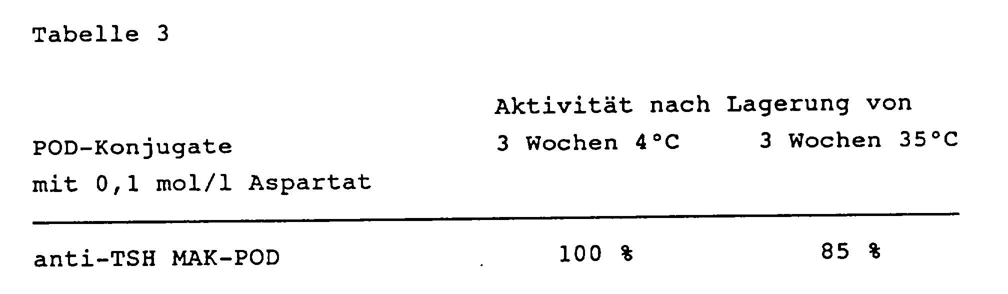

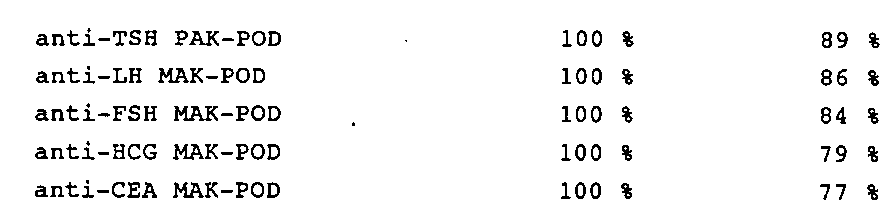

## Patentansprüche

1. Lagerfähige POD-Konjugatlösung,
**gekennzeichnet durch**
einen pH-Wert von 5,0 bis 6,5, eingestellt durch eine organische Puffersubstanz einer Konzentration von 40 bis 100 mmol/l und 0,05 bis 1,0 mol/l Magnesiumaspartat.

2. Lösung nach Anspruch 1,
**dadurch gekennzeichnet,**
daß sie als Puffersubstanz Good-Puffer, Citratpuffer und/oder 2-(N-Morpholino)-ethansulfonsäure (MES) enthält.

3. Lösung nach Anspruch 2,
**dadurch gekennzeichnet,**
daß sie als Puffer MES enthält.

4. Lösung nach einem der Ansprüche 1 bis 3,
**dadurch gekennzeichnet,**
daß sie zusätzlich Stabilisatoren, Konservierungsmittel und/oder inerte Proteine enthält.

5. Lösung nach Anspruch 4,
**dadurch gekennzeichnet,**
daß sie 0 bis 0,4 g/l Stabilisatoren enthält.

6. Lösung nach Anspruch 4 oder 5,
**dadurch gekennzeichnet,**
daß sie 0 bis 1 g/l Konservierungsmittel enthält.

7. Lösung nach einem der Ansprüche 4 bis 6,
**dadurch gekennzeichnet,**
daß sie 0 bis 20 g/l inertes Protein enthält.

## Claims

1. Storable POD conjugate solution, **characterised by** a pH value of from 5.0 to 6.5 adjusted by means of an organic buffer substance with a concentration of from 40 to 100 mmol/l and from 0.05 to 1.0 mol/l magnesium aspartate.

2. Solution according to claim 1, **characterised in that** it contains Good buffer, citrate buffer and/or 2- (N- morpholino)-ethanesulphonic acid (MES) as a buffer substance.

3. Solution according to claim 2, **characterised in that** it contains MES as a buffer.

4. Solution according to any of claims 1 to 3, **characterised** **in that** it additionally contains stabilisers, preserving agents and/or inert proteins.

5. Solution according to claim 4, **characterized in that** it contains 0 to 0.4 g/l of stabilisers.

6. Solution according to claim 4 or 5, **characterized in that** it contains 0 to 1 g/l of preserving agents.

7. Solution according to any of claims 4 to 6, **characterized** **in that** it contains 0 to 20 g/l of inert protein.

## Revendications

1. Solution de conjugué de POD stable au stockage, caractérisée par un pH de 5,0 à 6,5, établi par une substance tampon organique d'une concentration de 40 à 100 mmol/l et 0,05 à 1,0 mol/l d'aspartate de magnésium.

2. Solution selon la revendication 1, caractérisée en ce qu'elle contient comme substance tampon du tampon de Good, du tampon citrate et/ou de l'acide 2-(Nmorpholino) éthanesulfonique (MES).

3. Solution selon la revendication 2, caractérisée en ce qu'elle contient MES comme tampon.

4. Solution selon l'une des revendications 1 à 3, caractérisée en ce qu'elle contient en outre des stabilisants, des conservateurs et/ou des protéines inertes.

5. Solution selon la revendication 4, caractérisée en ce qu'elle contient 0 à 0,4 g/l de stabilisants.

6. Solution selon la revendication 4 ou 5, caractérisée en ce qu'elle contient 0 à 1 g/l de conservateurs.

7. Solution selon l'une des revendications 4 à 6, caractérisée en ce qu'elle contient 0 à 20 g/l de protéine inerte.
